# EUROPEAN PATENT APPLICATION

(11) **EP 1 882 486 A1**
(43) Date of publication of application: **30.01.2008**
(21) Application number: 07380200.1
(22) Date of filing: 05.07.2007
(51) Int. Cl.: A61M 5/165, A61M 5/31, A61M 5/50, A61J 1/14, G09F 3/02

(54) **Device for handling perfusion liquids with membrane filter and anti-tamper means**

(30) Priority: 28.07.2006 ES 200602044
(71) Applicant: Grifols, S.A., 08005 Barcelona (ES)
(72) Inventor: Girbau Campdelacreu, Joan, 08400 Granollers (Barcelona) (ES); Menendez Cortina, Jordi, 08041 Barcelona (ES)
(74) Representative: Durán Moya, Luis-Alfonso

(57) **Abstract**

The device comprises a container with a manually displaceable plunger, the container being extended, forming an integral assembly, a connection piece and a tubular element which has a filling opening at its end, the tubular element having an intermediate membrane filter for preventing the entry of harmful germs, the device also having anti-tamper security means in the portion disposed between the outlet from the container for the infusion liquid and the filter.

## Description

The present invention relates to a device for handling perfusion liquids which has characteristics of novelty and inventive step with respect to the prior art.

At the moment, syringes are used in which, once the measured filling has been performed, the liquid they contain will be used for direct perfusion into the human body or for incorporation with venous solution in a bag, bottle or infuser system for subsequent infusion.

Owing to the intended use of the solution which the syringe contains, that is, infusion into the human body, it is very important to maintain the sterility of the liquid contained until the moment of use.

One of the usual methods for the preparation of injectable medicines in a hospital pharmacy is the measured filling of the syringes, starting with injectable medicines in solution or reconstituted powders.

After preparation, the contents of the syringes are injected into a container of intravenous solutions (a bag, bottle, infuser, etc.) in the hospital pharmacy itself or are sent to the hospital ward for injection directly via a direct access route into the patient or injection into a connector connected to the patient (a bag, bottle, infuser, infusion pump equipment, etc.).

The syringe preparation process involves a degree of risk of contamination of the solution due to the handling that is performed (opening of containers, positioning of the needle, extraction of the solutions, reconstitution of powders and of lyophilized substances and subsequent extraction, etc.) and it is very important to be able to reduce this risk of contamination during preparation.

A possible reduction in the risk mentioned is achieved by the use of a filter for the aspiration or measuring of the liquid. However, it still constitutes an open system with the consequent risk of contamination.

The present invention is intended to solve the problem mentioned, enabling complete asepsis to be achieved in perfusion liquids.

To achieve its objective, the present invention discloses a device for handling perfusion liquids which incorporates integrally, and in a manner such that it cannot be dismantled without breaking a tamperproof element, a container for the liquid to be perfused, a tubular extension for the inlet and outlet of the liquid and a filter fitted between the liquid-inlet opening and the liquid container so as almost completely to prevent contamination of the liquid held in the container of the device, which may be welded, forming a single leaktight element, or may be connected to a hermetic connection also formed in a leaktight manner.

The filter included is a high efficiency filter with pores of the order of fractions of a micron, particularly of the order of 0.2 microns, the entire assembly of the device being sterilized by a suitable method so that its filling with the perfusion solution cannot add any contamination, given the arrangement of the filter fitted in the inlet duct.

It should be pointed out that, with the device of the invention, the device can be filled in an area which is not necessarily sterile since the measured filling of the device through the filter ensures the sterilization of the solution.

The characteristics of the filter which is made of a membrane with very small pore dimensions, enable its integrity to be tested after use, which provides the assurance that the liquid prepared for infusion has received correct filtration and therefore elimination of germs.

The device may also comprise a security means between the container and the tubular portion carrying the filter for indicating whether the device has been tampered with prior to its use.

Moreover, the present invention also provides for the security means optionally to be constituted by a tamper-proof label which has to be broken to disconnect the container from the rest of the device thus indicating any possible tampering. The label may be constituted by a laminar element provided with a line of weakening, by breakable welding between the container and the rest of the device, or by means of a heat-shrinkable label or a label of another type.

Some drawings of preferred embodiments of the present invention are appended by way of non-limiting example, for a better understanding thereof.

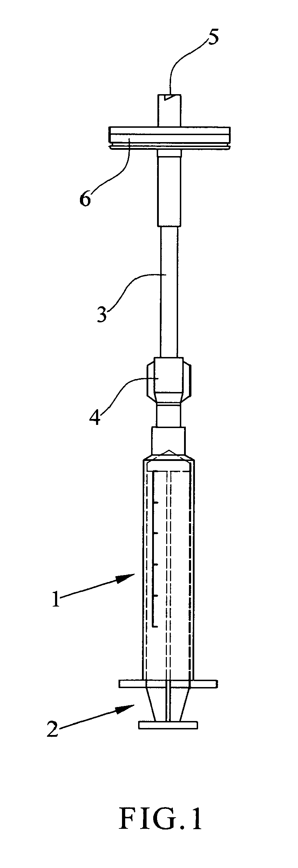

Figure 1 is a side elevational view of a device for handling perfusion liquids according to the present invention.
Figure 2 is an elevational view of a device similar to that of Figure 1 in the condition in which it is filled with solution, showing a closure by welding.
Figure 3 is a view similar to Figure 1 with a tamper-proof closure in the open position.
Figure 4 is a view similar to Figure 1 with a tamper-proof label.
Figure 5 is a detailed view of a tamper-proof label that can be applied to the device.
Figures 6 and 7 are respective views similar to Figure 1 showing a breakable weld and a heat-shrinkable label, respectively, as solutions for tamper-proof seals.

As will be appreciated from the drawings, the device for handling perfusion liquids of the present invention is composed integrally of a container 1 which is provided with a manual actuating plunger 2 and which is extended by a tubular element 3, by means of a connection piece 4, and terminates in an opening 5 for the filling of the container 1, a membrane filter 6 being provided in an intermediate position.

By virtue of this arrangement, it is practically impossible for harmful germs to enter the container 1 for the liquid to be perfused, which will be used for the filling of a perfusion container or for direct perfusion by means of a needle connected to the container. The filter 6 will have porosity of the order of fractions of a micron, particularly of the order of 0.2 microns.

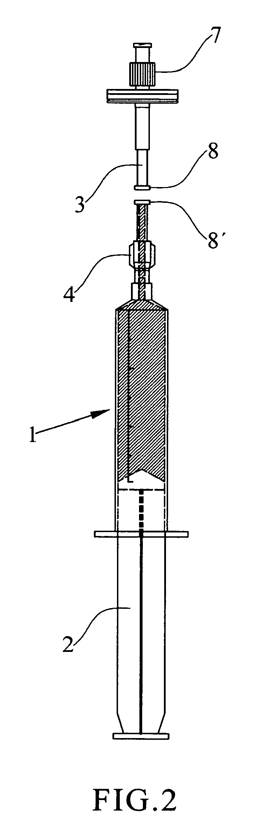

The terminal opening 5 may optionally have a stopper 7 and the tubular element 3 may have a closure by welding, of which the ends 8 and 8' are shown. Figure 2 shows the plunger 2 displaced along the cylinder 1, the filling of perfusion liquid inside the container 1 being represented by hatching.

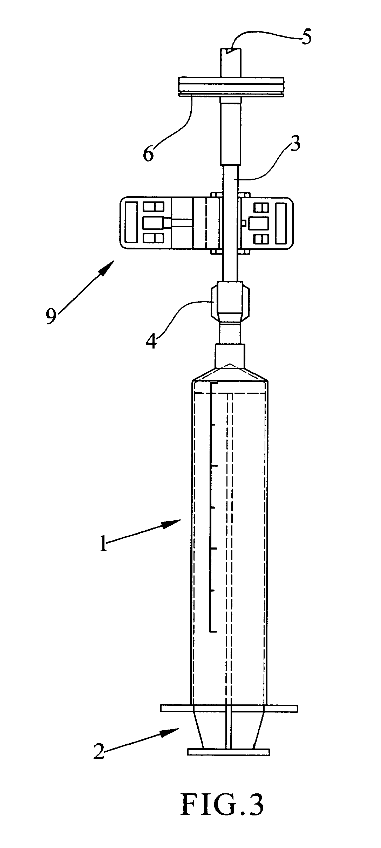

A tamper-proof closure 9, for example of the push-down type, may optionally be provided, as shown in Figure 3.

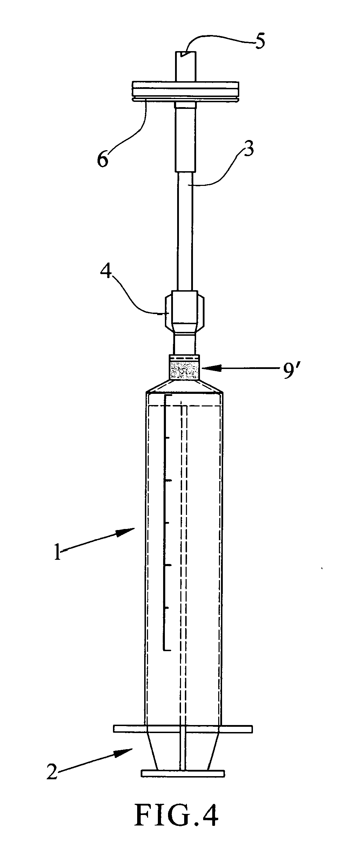

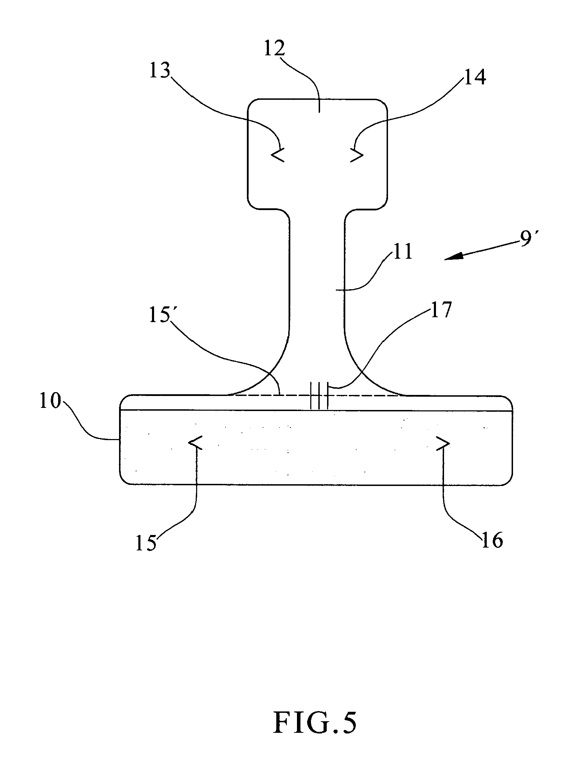

To improve the safety features of the device, it may be provided with a tamper-proof label 9', Figures 4 and 5, of a laminar type which fits the outlet of the container 1. The laminar label 9' may be constituted, as can be seen from Figure 5, by a substantially rectangular region 10 which can fit the outlet neck of the container 1 and a projecting stalk 11 with an extension 12 for joining it to the device, with incisions 13 and 14 as well as 15 and 16 for preventing complete detachment of the label. The label will comprise a tear line, indicated 15' in Figure 5, for the disconnection of the container from the rest of the device, and some printed traces or lines 17 which extend over the portion above and the portion below the line 15' to prevent tampering.

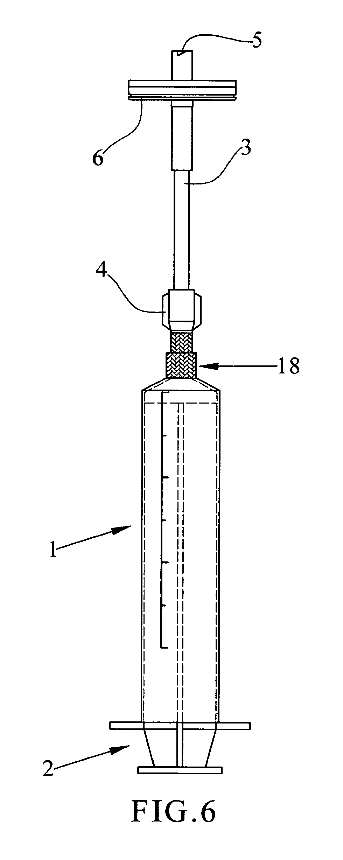

For tamper-proofing purposes, a breakable weld 18 may also be provided, extending over the outlet end of the container 1 and its neck or extension which is joined to the connection piece 4, Figure 6.

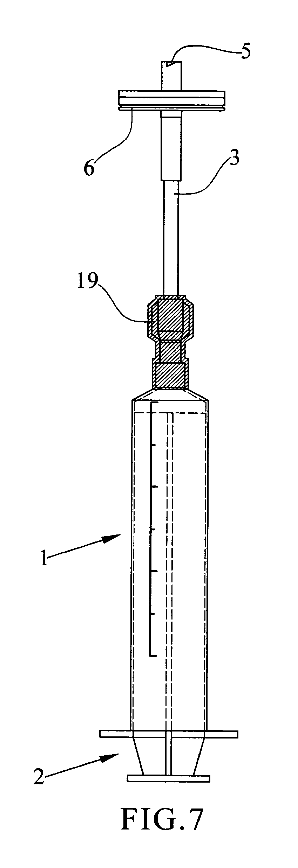

A heat-shrinkable label 19, which fits closely on the assembly, as can be seen in Figure 7, may also be provided, extending around the end region of the container 1 and the connection region 4.

As will be appreciated, the present invention provides substantial features of novelty and inventive activity with respect to the prior art, drastically reducing the possibility of contamination of the infusion liquid and incorporating means for indicating tampering with the syringe prior to its use by welding of a portion of the tubular outlet element of the container or by the use of suitable labels.

Although the invention has been described with reference to preferred embodiments, these should not be considered as limiting of the invention which will be defined by the broadest interpretation of the appended claims.

## Claims

1. Device for handling perfusion liquids, **characterized in that** it comprises a container with a manually displaceable plunger, the container being extended, forming an integral assembly, by a connection piece and a tubular element which has a filling opening at its end, which tubular element has an intermediate membrane filter for preventing the entry of harmful germs, the device also having anti-tamper security means in the portion disposed between an outlet from the container for the infusion liquid and the filter.

2. Device for handling perfusion liquids according to Claim 1, **characterized by** the provision of a breakable welded connection piece between the outlet of the container and the tubular element for connection to the filter.

3. Device for handling perfusion liquids according to Claim 1, **characterized by** the provision of a welded connection in the tubular element to ensure tamperproofness.

4. Device for handling perfusion liquids according to Claim 1, **characterized by** a heat-shrinkable label which covers the outlet of the container and the welded connection piece for anti-tamper purposes.

5. Device for handling perfusion liquids according to Claim 1, **characterized by** the provision of a label which extends around the outlet end of the container and which has a fitting region that extends around the end of the container with an extension that can be fixed to the device, a line of weakening existing between the rectangular, fitted portion of the label and the extension region thereof.

6. Device for handling perfusion liquids according to Claim 5, **characterized in that** the label has check lines extending across the line of weakening of the label for anti-tamper purposes.

7. Device for handling perfusion liquids according to Claim 6, **characterized in that** the label has incision regions in the rectangular portion and in the extension which can be fixed to the tubular element for connection to the filter to prevent complete detachment of the label, for anti-tamper purposes.
